(19) **Europäisches Patentamt / European Patent Office / Office européen des brevets**

(11) **EP 3 724 356 B1**

(12) **EUROPEAN PATENT SPECIFICATION**

(45) Date of publication and mention
of the grant of the patent:
**24.01.2024 Bulletin 2024/04**

(21) Application number: **18808349.7**

(22) Date of filing: **26.11.2018**

(51) International Patent Classification (IPC):
**C12Q 1/6886** *(2018.01)*

(52) Cooperative Patent Classification (CPC):
**C12Q 1/6886; C12Q 2600/178**

(86) International application number:
**PCT/EP2018/082563**

(87) International publication number:
**WO 2019/115214 (20.06.2019 Gazette 2019/25)**

(54) **BIOMARKERS FOR DISEASE BURDEN OF NEUROBLASTOMA**

BIOMARKER FÜR DIE ERKRANKUNGSBELASTUNG VON NEUROBLASTOM

BIOMARQUEURS DE CHARGE DE MALADIE DE NEUROBLASTOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2017 EP 17207577**

(43) Date of publication of application:
**21.10.2020 Bulletin 2020/43**

(73) Proprietor: **Universiteit Gent**
**9000 Gent (BE)**

(72) Inventors:
• **DECOCK, Anneleen**
**9681 Nukerke (BE)**
• **VANDESOMPELE, Joke**
**9870 Zulte (BE)**
• **ZEKA, Fjoralba**
**9000 Gent (BE)**
• **MESTDAGH, Pieter**
**8000 Brugge (BE)**

(56) References cited:
**WO-A1-2016/081941 WO-A2-2010/014975**

• **Satish Kumar Ramraj ET AL: "Serum-circulating miRNAs predict neuroblastoma progression in mouse model of high-risk metastatic disease", Oncotarget, 5 April 2016 (2016-04-05), page 18605, XP055478046, United States Retrieved from the Internet: URL:https://www.ncbi.nlm.nih.gov/pmc/artic les/PMC4951313/pdf/oncotarget-07-18605.pdf**
• **FAIZAN H KHAN ET AL: "Reorganization of metastamiRs in the evolution of metastatic aggressive neuroblastoma cells", BMC GENOMICS, BIOMED CENTRAL, vol. 16, no. 1, 7 July 2015 (2015-07-07), page 501, XP021225538, ISSN: 1471-2164, DOI: 10.1186/S12864-015-1642-X**
• **P. MESTDAGH ET AL: "High-throughput stem-loop RT-qPCR miRNA expression profiling using minute amounts of input RNA", NUCLEIC ACIDS RESEARCH, vol. 5-6,36, no. 21, 21 October 2008 (2008-10-21), pages e143-e143, XP055003045, ISSN: 0305-1048, DOI: 10.1093/nar/gkn725**
• **M. J. MURRAY ET AL: "Solid Tumors of Childhood Display Specific Serum microRNA Profiles", CANCER EPIDEMIOLOGY, BIOMARKERS AND PREVENTION., vol. 24, no. 2, 1 February 2015 (2015-02-01), pages 350-360, XP055255104, US ISSN: 1055-9965, DOI: 10.1158/1055-9965.EPI-14-0669**

• **Fjoralba Zeka ET AL: "Circulating microRNA biomarkers for metastatic disease in neuroblastoma patients", bioRxiv, 27 April 2018 (2018-04-27), XP055477658, DOI: 10.1101/309823 Retrieved from the Internet: URL:https://www.biorxiv.org/content/early/ 2018/04/27/309823.full.pdf**

Remarks:

The complete document including Reference Table(s) and the Sequence Listing(s) can be downloaded from the EPO website

**Description**

Technical field of invention

The present invention relates to monitoring disease progression. More in particular, the present invention discloses serum/plasma miRNA markers for assessment of disease burden in human neuroblastoma. Indeed, the present invention discloses that the more the tumor is spread throughout the body, the higher the levels of particular miRNAs in serum/plasma are. Consequently, the latter markers can be used in methods and kits for assessing the neuroblastoma disease burden.

Background art

Neuroblastoma is a childhood cancer arising from the developing sympathetic nervous system.[1] The disease is responsible for 11% of pediatric cancer deaths and is ranked third amongst cancers with the highest mortality rate.[2] Approximately 60% of the neuroblastoma patients are diagnosed with metastatic disease, mostly represented in the high-risk patient sub-group. High-risk patients have low survival rates of about 50%, and 2 thirds of these children will ultimately relapse, eventually entering a second line of intensive treatment, enrolling in an early-stage clinical trial or receiving mild palliative therapy.[1] Monitoring of treatment response is essential for assessment of its efficacy and for guiding the procedure of follow-up treatment after refractory or relapsed neuroblastoma.

Apart from non-molecular imaging methods, current methods for monitoring disease and evaluation of treatment response are mostly based on detection of minimal residual disease (MRD), which is determined by estimating the number of residual tumor cells in peripheral blood or bone marrow[3]. While several cellular MRD markers have been proposed, biomarkers for non-invasive cell-free determination of disease burden are currently lacking.

MicroRNAs, a class of small non-coding RNAs involved in various aspects of health and disease, including neuroblastoma pathophysiology[4], seem to be particularly stable and abundant in serum and plasma[5]. Some studies have reported a relationship between serum miRNAs and disease characteristics such as *MYCN* amplifications, or increased risk for adverse outcome[7]. However, there is still a need to identify and select specific markers which are found preferably in liquid biopsies and which can be used to determine the disease burden in neuroblastoma.

Brief description of figures

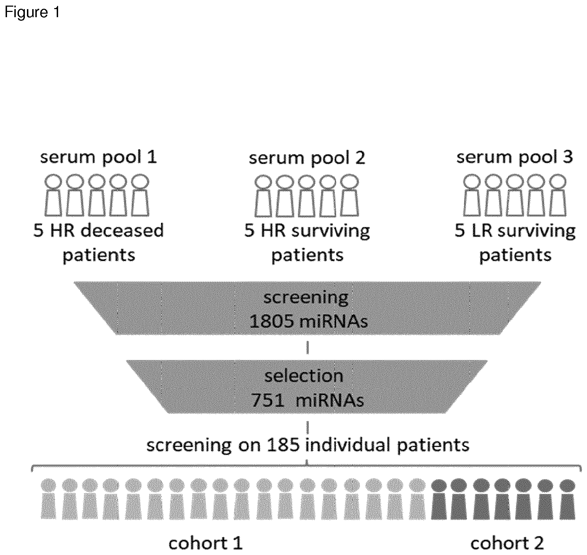

Figure 1: Three serum pools were prepared, each containing 5 serum samples from three neuroblastoma sub-groups: 5 high-risk (HR) deceased patients, 5 high-risk surviving patients and 5 low-risk (LR) surviving patients for serum pool 1, 2 and 3, respectively. Expression of 1805 miRNAs was measured by RT-qPCR. 751 well-expressed miRNAs were selected and profiled on two independently collected and processed patient cohorts of 131 patients and 54 patients, respectively.

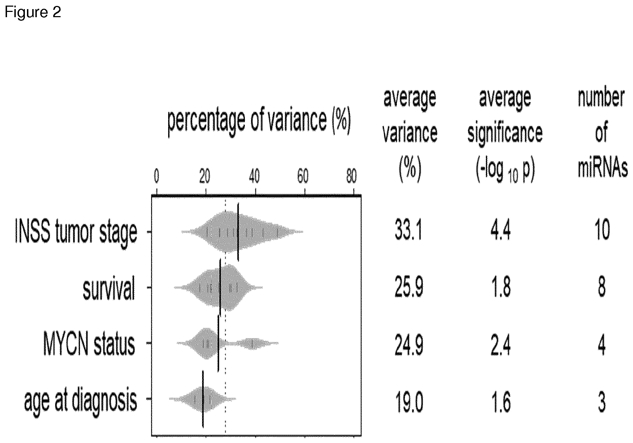

Figure 2: Bean plot of the average variance per disease feature (%), average significance (-log 10 of the P-value) and number of miRNAs significantly influenced by disease features. Gray lines in the violin plot represent percentage of variance for individual miRNAs. The evaluated disease features were tumor stage (INSS tumor stage), overall survival (survival), *MYCN* status and age at diagnosis. P-values were corrected for multiple-testing error by the Benjamini-Hochberg's-method prior to averaging.

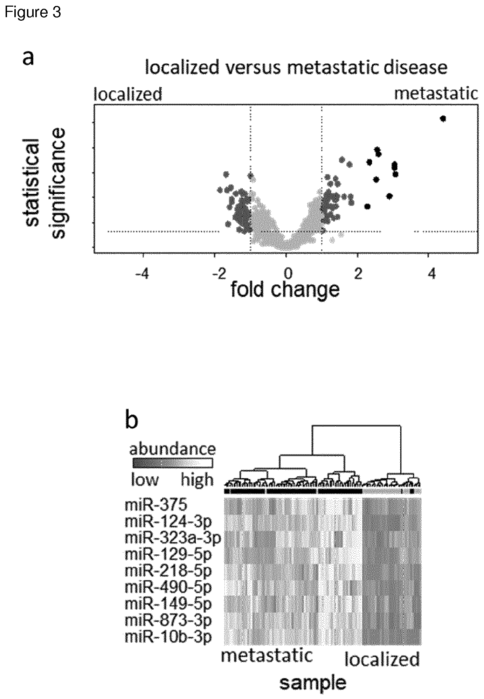

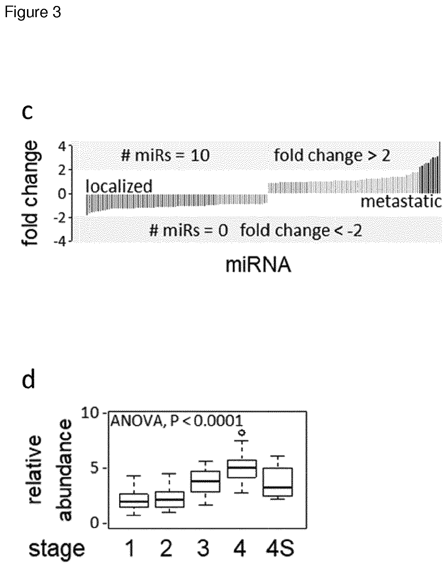

Figure 3: a) Fold change (log2 scale) of miRNA abundance in serum (x-axis) against statistical significance (y-axis, -log10 of the P-value) between patients with metastatic (stage 4) neuroblastoma (n=76) and patients with localized (stage 1 and stage 2) neuroblastoma (n=33). Higher fold change denotes higher abundance in metastatic serum. Dark gray dots represent miRNAs with at least two-fold (1 log2 unit) higher (right) or lower (left) abundance and black dots represent miRNAs with at least four-fold higher abundance in serum from metastatic patients. For the selection of differentially abundant miRNAs, a Mann-Whitney U test was used at 0.05 significance level (corrected for multiple-testing by the Benjamini-Hochberg's method). b) Fold change distribution of miRNA abundance in localized versus metastatic serum. The bar colors correspond to dot colors in plot (a). c) Heatmap depicting hierarchical clustering of serum samples (cohort 1) based on 9 miRNAs with at least four-fold higher abundance in serum from metastatic patients. d) Boxplot of average miRNA expression for the selected set of 9 miRNAs in serum from stage 1, 2, 3, 4 and 4S patients.

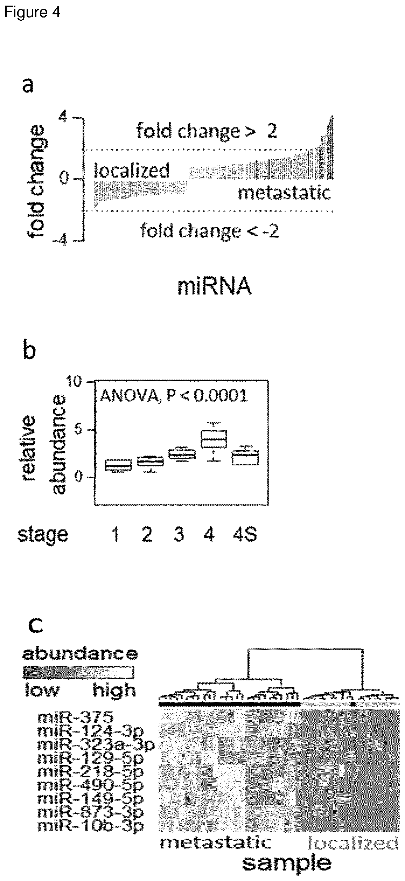

Figure 4: a) Fold change distribution of miRNA abundance between sera from metastatic (stage 4) neuroblastoma (n=27) and localized (stage 1 and 2) neuroblastoma (n=17) patients (cohort 2, n=54). Black bars indicate the position of the 9 miRNAs identified in the first cohort. **b)** Boxplot of miRNA abundance for the selected 9 miRNAs in sera from stage 1, 2, 3, 4 and 4S disease, in cohort 2. **c)** Heatmap depicting hierarchical clustering of serum samples based on selected set of 9 miRNAs.

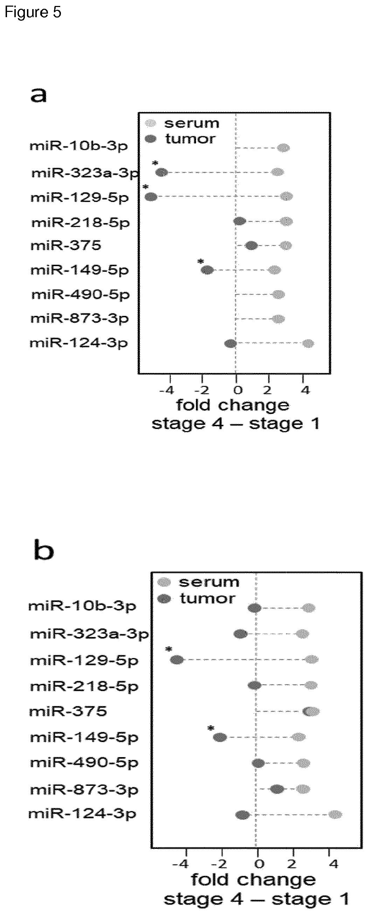

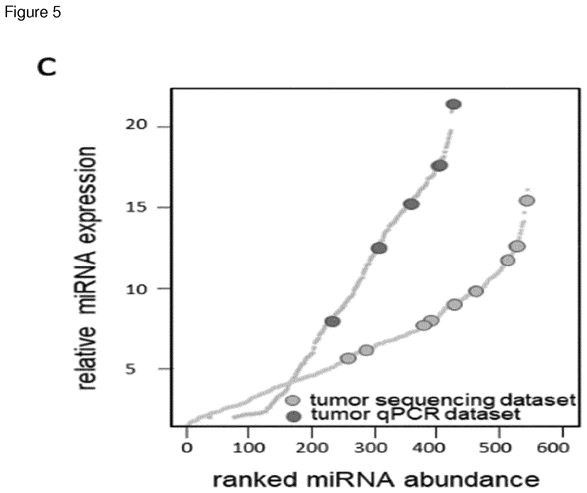

**Figure 5**: a) miRNA abundance differences in serum and in tumor tissue measured by stem-loop RT-qPCR. **b)** miRNA abundance differences in serum and in tumor tissue measured by small RNA sequencing. The serum markers for metastatic disease that are differentially expressed between stage 4 and stage 1 tumor are marked by asterix (Mann-Whitney U test P values <0.05, Benjamini-Hochberg correction for multiple testing); dots represent fold changes in tumor tissue (left) and serum (right). **c)** Ranked miRNA abundance in the RT-qPCR data and in the small RNA sequencing data.

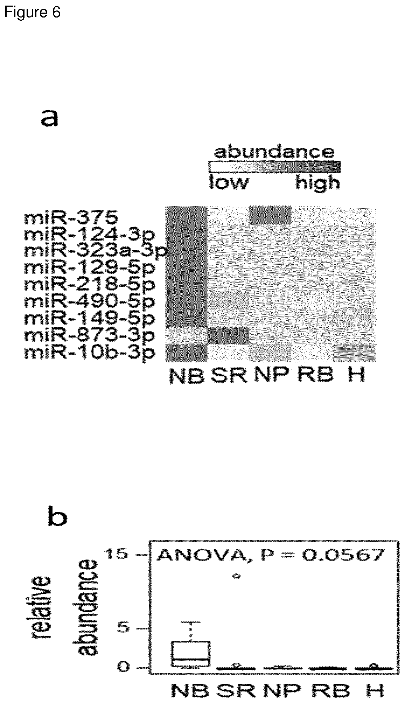

**Figure 6**: a) Standardized relative abundance for individual circulating miRNAs in serum pools from neuroblastoma (NB), sarcoma (SR), nephroblastoma (NP) rhabdomyosarcoma (RB) patients and healthy children (H). **b)** Relative abundance of circulating miRNAs in serum pools from neuroblastoma (NB), sarcoma (SR), nephroblastoma (NP) rhabdomyosarcoma (RB) patients and healthy children (H).

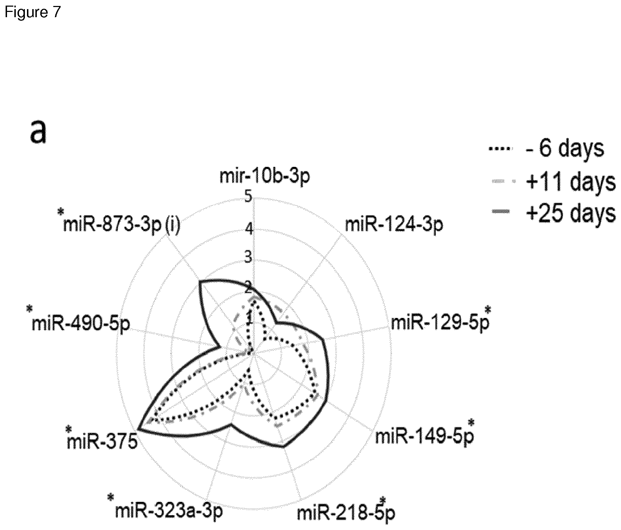

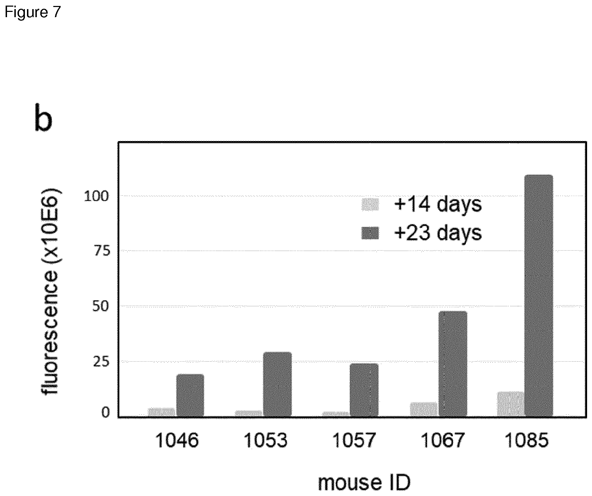

**Figure 7**: a) Average miRNA abundance (log10 normalized values) in murine serum from 8 engrafted mice, 6 days before engraftment, and 11 days and 25 days after engraftment. **b)** Luciferase fluorescence assessment of tumor volume, 14 days and 23 days after engraftment for 5/8 mice in panel (a). (i) miR-873-3p is a human specific miRNA assay. (*) significant increase of miRNA abundance over time based on linear mixed-effects model analysis (p<0.05).

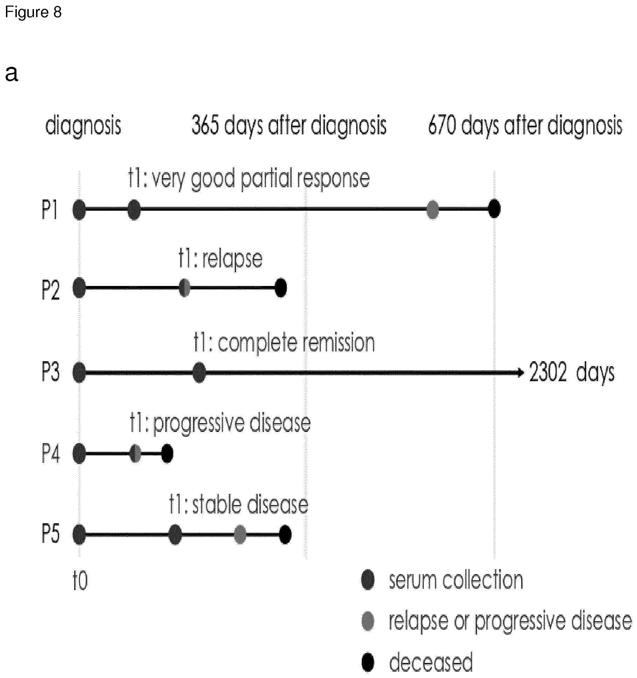

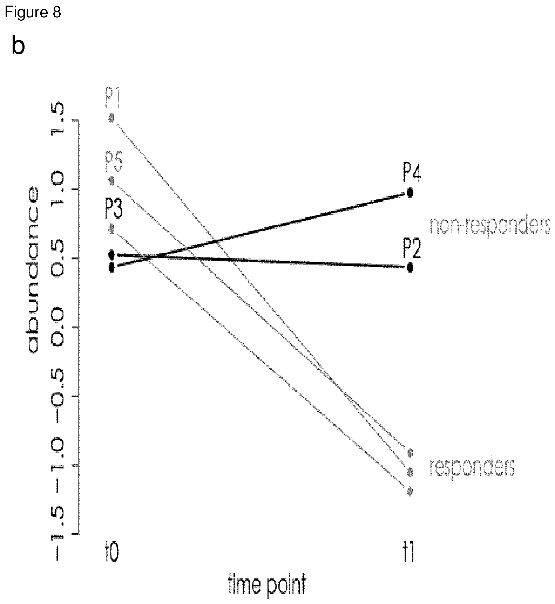

**Figure 8**: a) Serum abundance of disease burden miRNA markers changes during treatment of stage 4 neuroblastoma patients. **a)** The disease course for each of the patients (P1-P5) is depicted, including the time points of serum collection and occurrence of disease events (relapse, progressive disease and death). **b)** For each of the individual patients, the change in average abundance level of the 9 disease burden miRNA markers in serum is shown. Note that at time point t1, patient P1, P3 and P5 are considered treatment responders, while patient P2 and P4 are considered non-responders.

## Description of invention

The circulating miRNome from 185 diagnostic serum samples was quantified and the association of the four following disease characteristics with serum miRNA abundance was assessed: the international neuroblastoma staging system (INSS), gene copy number status of the *MYCN* oncogene, age at diagnosis, and overall survival. It was found that there exists a strong positive correlation between a proportional increase of a selection of 9 miRNAs with an increasing tumor stage. Indeed, evaluation of the selected set of 9 miRNAs in serum from stage 1, 2, 3, 4 and 4S disease showed that the more the disease is spread throughout the body, the higher the levels of the selected miRNAs in serum are. As tumor stage reflects how far the cancer has spread, this set of miRNAs can clearly be used as serum markers for assessment of disease burden in human neuroblastoma. It is further clear that the latter set of miRNAs can be used as serum markers in methods for monitoring disease and evaluation of treatment response.

Therefore, the present invention relates to an in vitro method for assessing the disease burden in human neuroblastoma patients comprising:

- providing a sample obtained from a patient,

- quantifying the expression level of the following miRNAs: miR-124-3p, miR-375, miR-323a-3p, miR-129-5p, miR-218-5p, miR-490-5p, miR-149-5p, miR-873-3p and miR-10b-3p in said sample, and,

- assessing the disease burden based on the quantity of the expression level of at least one of said miRNAs wherein a relative increased abundance of at least one of said miRNAs correlates with an increased disease burden, wherein said assessing the disease burden allows to monitor disease and to evaluate treatment response.

With the term 'disease burden' as used herein is meant the tumor volume (primary and/or metastatic lesions) or the size of the cancer measured by the amount of space taken up by the tumor (primary and/or metastatic lesions). The latter term further correlates with the disease stage -i.e. the stage of a cancer that describes how far the cancer has spread- according to the International Neuroblastoma Staging System. The latter Staging System is explained in detail in WO2010/066851. Hence, the terms 'disease burden', 'tumor volume ' and 'disease stage' can be used interchangeably.

The terms 'quantifying the expression level or abundance of at least one of the list consisting of the following miRNAs: miR-124-3p, miR-375, miR-323a-3p, miR-129-5p, miR-218-5p, miR-490-5p, miR-149-5p, miR-873-3p and miR-10b-3p in said sample' refer to any method known in the art which allows to determine the amount or abundance of at least one specific miRNA molecule which is part of the given list in a given sample volume. Non-limiting examples of the latter methods are PCR-based methods, hybridization-based methods or sequencing-based methods. Examples of PCR-based methods are PCR, RT-PCR, end-point PCR, digital PCR or the like as are described in detail in -for example- WO2010/066851. Non-limiting examples of hybridization based methods are microarray, digital gene expression (DGE), RNA-in-situ hybridization (RISH), Northern-blot analysis and the like as are described in detail in -for example- WO2010/066851. Non-limiting examples of sequencing-based methods are Supported Oligonucleotide Detection, Pyrosequencing, Polony Cyclic Sequencing by Synthesis, Simultaneous Bidirectional Sequencing, Single-molecule sequencing, Single molecule real time sequencing, True Single Molecule Sequencing, Hybridization-Assisted Nanopore Sequencing and Sequencing by synthesis as are described in detail in -for example-WO2010/066851.

The terms 'assessing the disease burden based on the quantity of the expression level of at least one of said miRNAs wherein a relative increased abundance of at least one of said miRNAs correlates with an increased disease burden' relates to the fact that the more the disease is spread throughout the body, the higher the levels of the selected miRNAs in serum are. For example, stage 4 patients having metastatic disease show a four-fold increase of the amount of the selected miRNAs in serum when compared to the amount which is found in stage 1 or stage 2 patients. Stage 4S patients (i.e. patients having metastatic disease with good outcome due to spontaneous regression) and stage 3 patients have a three-fold increase of the amount of the selected miRNAs in serum when compared to the amount which is found in stage 1 or stage 2 patients.

The terms "wherein said assessing the disease burden allows to monitor disease and to evaluate treatment response" indicates that the abundance of the miRNAs do not solely correlate with assessing risk levels of disease but rather with tumor load/disease burden. Indeed, the abundance of the miRNAs can initially be high in high-risk patients but said abundance may decrease after treating said patients with anti-tumor means. Hence, the miRNAs of the present invention do not solely assess risk levels but can be -surprisingly- used to monitor disease before, during and after treatment.

The miRNAs disclosed in the present invention, i.e. miR-375, miR-124-3p, miR-323a-3p, miR-129-5p, miR-218-5p, miR-490-5p, miR-149-5p, miR-873-3p and miR-10b-3p, are well-known miRNAs having the following MIMAT IDs (www.mirbase.org): miR-375 = MIMAT0000728, miR-124-3p = MIMAT0000422, miR-323a-3p = MIMAT0000755, miR-129-5p = MIMAT0000242, miR-218-5p = MIMAT0000275, miR-490-5p = MIMAT0004764, miR-149-5p = MIMAT0000450, miR-873-3p = MIMAT0022717 and miR-10b-3p = MIMAT0004556, or are isomiRs derived from the latter miRNAs.

More specifically, the present invention relates to an in vitro method as described above wherein said quantification of the expression level is undertaken on the following list of miRNAs: miR-375, miR-124-3p, miR-323a-3p, miR-129-5p, miR-218-5p, miR-490-5p, miR-149-5p, miR-873-3p and miR-10b-3p, and wherein said assessing the disease burden is based on the quantity of the expression level of all of said miRNAs and wherein a relative increased abundance of all of said miRNAs correlates with an increased disease burden.

In particular, the present invention relates to an in vitro method as described above wherein said sample obtained from a patient is a serum or a plasma sample. Furthermore, the present invention relates to an in vitro method as described above wherein said increased disease burden corresponds to stage 4 disease according to the International Neuroblastoma Staging System.

The present invention further relates to a method for assessing the disease burden in human neuroblastoma patients utilizing means for quantifying the expression level of the following miRNAs: miR-124-3p, miR-375, miR-323a-3p, miR-129-5p, miR-218-5p, miR-490-5p, miR-149-5p, miR-873-3p and miR-10b-3p.

With the terms 'means for quantifying the expression level' is for example meant a primer pair specific for each of the miRNAs for use in a PCR-based method, b) a probe specific for each of the miRNAs for use in a hybridization-based method or c) is any means suitable for use in sequence-based analysis of each of the miRNAs. Specific examples of the latter 'means for quantifying the expression level' can be designed based on the following nucleic acid sequence of each of the following miRNAs:

| name | MIMAT ID | Sequence | SEQ ID N° |
|------|----------|----------|-----------|
| miR-375 | MIMAT0000728 | uuuguucguucggcucgcguga | SEQ ID N° 1 |
| miR-873-3p | MIMAT0022717 | ggagacugaugaguucccggga | SEQ ID n° 2 |
| miR-149-5p | MIMAT0000450 | ucuggcuccgugucuucacuccc | SEQ ID N° 3 |
| miR-124-3p | MIMAT0000422 | uaaggcacgcggugaaugcc | SEQ ID N° 4 |
| miR-218-5p | MIMAT0000275 | uugugcuugaucuaaccaugu | SEQ ID N° 5 |
| miR-490-5p | MIMAT0004764 | ccauggaucuccaggugggu | SEQ ID N° 6 |
| miR-323a-3p | MIMAT0000755 | cacauuacacggucgaccucu | SEQ ID N° 7 |

(continued)

| name | MIMAT ID | Sequence | SEQ ID N° |
|---|---|---|---|
| miR-10b-3p | MIMAT0004556 | acagauucgauucuaggggaau | SEQ ID N° 8 |
| miR-129-5p | MIMAT0000242 | cuuuuugcggucugggcuugc | SEQ ID N° 9 |

More specifically, the present invention relates to a method for assessing the disease burden in human neuroblastoma patients utilizing means for quantifying the expression level of the following list of miRNAs: miR-375, miR-124-3p, miR-323a-3p, miR-129-5p, miR-218-5p, miR-490-5p, miR-149-5p, miR-873-3p and miR-10b-3p.

Furthermore, the present invention relates to a method as described wherein said means for quantifying the expression level of miRNAs is a) a primer pair specific for each of the miRNAs for use in a PCR-based method, b) a probe specific for each of the miRNAs for use in a hybridization-based method or c) is any means suitable for use in sequence-based analysis of each of the miRNAs.

The present invention will further be illustrated by the following non-limiting examples.

<u>Examples</u>

**Materials and methods**

**Patient demographics**

Serum was collected from 185 neuroblastoma patients at diagnosis from 6 different clinical centers: Ghent (Belgium), Essen and Cologne (Germany), Lyon (France), Tel Aviv (Israel), and Brno (Czech Republic). The serum samples were collected and processed in two independent cohorts. Cohort 1 contained 131 samples from all six centers; cohort 2 contained 54 samples from Cologne. Serial serum samples, collected at diagnosis (t0) and during treatment (t1), from 5 patients diagnosed with metastatic disease (stage 4) neuroblastoma were collected at the Centre Léon Bérard (Lyon, France). Serum from healthy children (5 samples) and diagnostic serum for three additional cancer entities (sarcoma, nephroblastoma and rhabdomyosarcoma, 5 samples each) were obtained from Ghent University Hospital, Ghent, Belgium.

**Serum sample pooling**

Three neuroblastoma serum pools were prepared from human diagnostic samples (Table 1), each containing 200 $\mu$l from 5 serum samples (40 $\mu$l each): pool 1 contained 5 high-risk patients that died of the disease within 36 months after diagnosis; pool 2 contained 5 high-risk surviving patients with at least 5 years follow-up time; pool 3 contained 5 low-risk surviving patients with at least 5 years follow-up time.

Table 1: Clinico-pathological properties of the serum samples used to prepare the three serum pools composed of 5 high-risk patients that died of the disease within 36 months after diagnosis, 5 high-risk surviving patients with at least 5 years follow-up time and 5 low-risk surviving patients with at least 5 years follow-up time, respectively.

| patient ID | INSS stage | MYCN status (0=non-amplified; 1=amplified) | age at diagnosis (m) | event-free survival (m) | event-free survival 2 (0=no; 1=yes) | over-all survival (m) | overall survival 2 (0=no; 1=yes) | risk class (0=no; 1=yes) |
|---|---|---|---|---|---|---|---|---|
| 982962 | 4 | 1 | 77,1 | 13,8 | 1 | 17,2 | 1 | HR1 |
| K05/5174 | 4 | 1 | 35,6 | 15,9 | 1 | 18,1 | 1 | HR1 |
| 302960 | 5 | 1 | 3,2 | 2,6 | 1 | 14 | 1 | HR1 |
| 302934 | 4 | 0 | 19,7 | 8,3 | 1 | 15,1 | 1 | HR1 |
| K05/0894 | 4 | 1 | 57,4 | 24,8 | 1 | 24,8 | 1 | HR1 |
| K05/4401 | 4 | 0 | 55,6 | 76,6 | 0 | 76,6 | 0 | HR0 |
| 25851 | 4 | 1 | 77,5 | 64,6 | 0 | 64,6 | 0 | HR0 |
| 410821 | 2 | 1 | 14,4 | 158,4 | 0 | 158,4 | 0 | HR0 |

(continued)

| patient ID | INSS stage | MYCN status (0=non-amplified; 1=amplified) | age at diagnosis (m) | event-free survival (m) | event-free survival 2 (0=no; 1=yes) | over-all survival (m) | overall survival 2 (0=no; 1=yes) | risk class (0=no; 1=yes) |
|---|---|---|---|---|---|---|---|---|
| K05/4412 | 4 | 0 | 15,3 | 148,3 | 0 | 148,3 | 0 | IR0/HR0 |
| 54666 | 4 | 1 | 67,7 | 187,9 | 0 | 187,9 | 0 | HR0 |
| 302780 | 2 | 0 | 102,2 | 213 | 0 | 213 | 0 | LR0 |
| K05/4975 | 1 | 0 | 6,5 | 24,2 | 1 | 218,4 | 0 | LR0 |
| K05/4538 | 2 | 0 | 10,6 | 225,2 | 0 | 225,2 | 0 | LR0 |
| K05/4456 | 2 | 0 | 6,5 | 192,9 | 0 | 192,9 | 0 | LR0 |
| 65412 | 1 | 0 | 24,5 | 175,2 | 0 | 175,2 | 0 | LR0 |

The same approach was used for preparation of three additional pools from diagnostic serum from the other pediatric cancer entities and healthy controls.

**RNA isolation, reverse transcription and RT-qPCR reaction**

The serum pools were screened for 1805 microRNAs using qPCR (miRBase release version 20), as described in Zeka et al., 2015. The individual samples were screened for 754 microRNAs, including 751 human miRNAs and 3 spike-in control miRNAs: cel-miR-39-3p, miRTC (miRNA reverse transcription control, included in the RT kit) and PPC (positive PCR control, synthetic spike-in control included in the SYBR Green PCR mix). Briefly, the miRNeasy serum/plasma kit (Qiagen) was used to extract RNA from 200 $\mu$l serum. The serum samples were lysed with 1000 $\mu$l Qiazol and spiked with 3.5 $\mu$l synthetic cel-miR-39-3p ($1.6 \times 10^8$ copies/$\mu$l). 200 $\mu$l chloroform was used for phase separation at 4 °C and 12 000 g. 600 $\mu$l of the aqueous phase was transferred onto an RNeasy MiniElute spin column and subsequently washed with 100% ethanol, buffer RWT, buffer RPE and 80% ethanol. The total RNA fraction was eluted with 14 $\mu$l RNase-free water.

For reverse transcription (miScript II RT kit, Qiagen), 1.5 $\mu$l RNA was mixed with 1 $\mu$l reverse transcriptase, 2 $\mu$l RT buffer, 1 $\mu$l nucleic acid mix and 4.5 $\mu$l RNase-free water. Samples were incubated for 60 minutes at 37 °C and 5 minutes at 95 °C. 2 $\mu$l of the RT product was diluted 22-fold with 42 $\mu$l nuclease-free water and used for quantification of synthetic spike-in RNA molecules: cel-miR-39-3p and miRTC in 10 $\mu$l qPCR reactions. To this purpose, 2 $\mu$l of the diluted RT product was mixed with 5 $\mu$l SYBR Green PCR mix, 1 $\mu$l universal primer, 1 $\mu$l miRNA specific primer and 1 $\mu$l RNase-free water. Samples were incubated at 95 °C for 15 minutes, followed by 40 cycles of denaturation (15 seconds at 94 °C) annealing (30 seconds at 55 °C) and extension (30 seconds at 70 °C).

For the preamplification reaction (miScript PreAMP PCR Kit, Qiagen), 5 $\mu$l of 5-fold diluted RT product was mixed with 5 $\mu$l preamp buffer, 2 $\mu$l DNA polymerase, 5 $\mu$l target specific assay pool, 1 $\mu$l universal primer mix and 7 $\mu$l RNase-free water to obtain a 25 $\mu$l preamplification reaction. Samples were incubated at 95 °C for 15 minutes, followed two cycles of denaturation (30 seconds at 94 °C) annealing (60 seconds at 55 °C) and extension (60 seconds at 70 °C) and 10 additional cycles of denaturation (30 seconds at 94 °C) and one-step annealing/extension (3 minutes at 60 °C). 100 $\mu$l nuclease-free water was added to the 25 $\mu$l preamplification product in order to obtain a 5-fold dilution.

For miRNA quantification on the preamplified product, 5 $\mu$l qPCR reactions were prepared for each miRNA and each sample. 384-well plates, pre-spotted with 1X miRNA qPCR primer assays were used. qPCR mix was prepared for each multi-well plate by mixing 1025 $\mu$l SYBR Green PCR mix, 205 $\mu$l universal primer mix, 50 $\mu$l preamplified template cDNA, 770 $\mu$l nuclease-free water and dispensed by a Tecan Evo 75 liquid handler (5 $\mu$l per well). The cycling conditions for quantitative PCR consisted of an initial activation step (15 minutes at 95 °C) and 40 cycles of denaturation (15 seconds at 94 °C), annealing (30 seconds at 55 °C) and extension (30 seconds at 70 °C). The positive PCR control PPC was included as qPCR processing control.

All qPCR reactions were performed on a CFX384 Real Time PCR Detection System (Bio-Rad). Cq-values were determined by the CFX software manager version 3.1 with the regression Cq-value determination mode.

**Quality control of the RT-qPCR data**

Quantification of cel-miR-39-3p, added to the serum samples during lysis, was used as processing control of the serum RNA isolation. Two samples with no detection of cel-miR-39-3p were excluded from the study. MiRTC level differences between serum samples and negative control samples (water mixed with MS2 phage RNA) were used to evaluate RT efficiency and qPCR inhibition. 22 serum samples were excluded due to a miRTC Cq-value of 1 cycle higher than the negative control samples. PPC levels across all 384-plates were used to asses deviating global Cq shifts as result of inter-run variation, batch-effect or technical failure.

The Cq detection cut-off was determined based on evaluation of single positive signals obtained from replicate miRNA expression data, as described in Mestdagh et al. (2014).[8] In the current study, two neuroblastoma sample pools were used instead of technical replicate samples. The fraction of single positive results was reduced by 95% at Cq-value 29, which was chosen as Cq detection cut-off.

The qPCR reproducibility was assessed for 4 representative sequences in all tested samples: hsa-miR-1224-5p: endogenous human microRNA; cel-miR-39-p: synthetic C. elegans microRNA spiked into each serum sample to assess RNA extraction efficiency; miRTC: short synthetic RNA sequence that is spiked by the manufacturer into the reverse transcription master mix; PPC: short synthetic DNA sequence that is spiked by the manufacturer into the qPCR master mix. For 97% of the duplicate reactions, $\Delta$Cq values lower than 0.5 were observed, indicating a high RT-qPCR repeatability.

**Collection and processing of murine serum samples**

miRNA expression profiles from murine serum were generated through small RNA sequencing of serum samples from eight female mice bearing orthotopic neuroblastoma xenografts and eight control (non-engrafted) female mice. 1 $\times$ 10$^6$ human luciferase-SH-SY5Y neuroblastoma cells were surgically implanted in athymic immunodeficient NCr nude mice under the subrenal capsule. Tumor size was assessed 14 days and 23 days after injection of neuroblastoma cells, by measurement of luciferase intensity. 100 $\mu$l blood was collected, 6 days before, 11 days after and 25 days after tumor cell injection in serum BD Vacutainer tubes. 50 $\mu$l of the resulting serum was used for RNA isolation with the miRNeasy serum/plasma kit (Qiagen). Subsequently, tRNA fragment depletion was performed as described in Van Goethem et al. (2016)[9]. The tRNA depleted RNA samples were then suspended in 7.5 $\mu$l RNAse-free water and used for small RNA library preparation by TruSeq small RNA library preparation kit v2 (Illumina). Library size selection was performed on a Pippin Prep (Sage Science) device with a specified collection size range of 125-153 bp. Libraries were further purified and concentrated by precipitation and quantified using qPCR. Subsequently, equimolar library pools were prepared, and further diluted to 4 nM. The pooled library was sequenced at a final concentration of 1.2 pM on a NextSeq 500 using high output v2 kits (single-end, 75 cycles, Illumina). Quantification of small RNAs was done using Cobra, Biogazelle's small RNA seq data analysis pipeline. For publically available alternatives, we advise the use of miRDeep2 and miRExpress.

**Processing of the serial neuroblastoma serum samples**

RNA from the serially collected serum samples (200 $\mu$l) was extracted using the miRNeasy serum/plasma kit (Qiagen). Additionally, 10 RNA samples from cohort 1 that have the highest and lowest average abundance level of the 9 disease burden miRNA markers, were reprofiled, to serve as control samples. miRNA abundance levels of these samples were profiled by RT-qPCR, using miScript Primer Assays (Qiagen) for the 9 disease burden miRNA markers (miR-375 (MS00031829), miR-873-3p (MS00037835), miR-149-5p (MS00003570), miR-124-3p (MS00006622), miR-218-5p (MS00006769), miR-490-5p (MS00009786), miR-323a-3p (MS00037219), miR-10b-3p (MS00008421) and miR-129-5p (MS00006643)) and 8 reference miRNAs (miR-3909 (MS00023513), miR-1203 (MS00014154), miR-4800-5p (MS00038962), miR-4755-5p (MS00039417), miR-877-3p (MS00010654), miR-1976 (MS00016751), miR-766-3p (MS00005439) and miR-532-3p (MS00010052)).

RT and preamplification was performed as described in Zeka et al., 2015. For preparation of the miScript PreAmp Primer Mix, 70 $\mu$l of RNase-free water was mixed with 10 $\mu$l miRTC miScript Primer Assay and 10 $\mu$l of each of the 10x target miScript Primer Assays (diluted in 550 $\mu$l TE, pH 8.0).

miRNA quantification was performed in 10 $\mu$l qPCR reactions, consisting of 5 $\mu$l SYBR Green PCR mix, 1 $\mu$l 10x miScript Universal Primer, 1 $\mu$l 10x target miScript Primer Assay, 1 $\mu$l nuclease-free water and 2 $\mu$l 5-fold diluted preamplification product. qPCR reactions were performed on a LightCycler 480 (LC480) system (Roche). Cycling conditions were as described above. Cq-values were determined by the LC480 software, using the second derivative maximum method.

**Preprocessing and data analysis**

In order to define the neuroblastoma serum miRNome, pairwise comparisons of miRNA expression of the three serum pools was performed to select miRNAs detected in at least one of the serum pools based on three selection criteria. First, miRNAs with ΔCq larger than 1.5 between two pools were selected (303 miRNAs). Second, miRNAs showing Cq-values below 27 in one sample and an absent signal (Cq > 29) in the other sample were selected (90 miRNAs). The final selection criterion was based on detection (Cq < 28) in the high-risk serum pools (358). This resulted in a total of 751 miRNAs that were subsequently profiled in a first patient cohort of 131 individual serum samples. A second patient cohort containing 54 neuroblastoma serum samples was independently collected in a later stage of the study and processed by a different batch of miRNA expression profiling reagents.

A threshold Cq-value of 29 was determined based on the evaluation of single positive signals obtained from replicate miRNA expression data, as described in Mestdagh et al. (2014).[8]

After removal of Cq-values above 29 cycles, miRNAs with missing values in more than 75% (an empirically chosen threshold) of the samples were excluded from the dataset. This led to exclusion of 8 miRNAs.

Raw Cq data were normalized as described in D'haene et al., (2012).[10] Briefly, the arithmetic mean Cq for each miRNA across all samples was determined and subtracted from individual Cq-values (i.e. mean centering of the miRs). Then, the arithmetic mean Cq value per sample was calculated across all miRNAs and subtracted from each individual Cq-value to obtain normalized miRNA expression values per sample.

Generalized additive modeling, Man-Whitney U test, Student's T-test and Analysis of variances (ANOVA) was performed using R statistical programing tool version 3.3.2.

For the evaluation of the variance of miRNA abundance in serum by GAM analysis[11], the following mathematic model was used:

$$\textit{miRNA expression}_i = \textit{ß}_1 * \textit{INSS tumor stage} + \textit{ß}_2 * \textit{MYCN status} + \textit{ß}_3 * \textit{survival} + \textit{ß}_4 * \textit{age at diagnosis}$$

*For: i = miRNA 1 to miRNA 743*

*$\beta$ (1 to 4) = coefficient of the prediction model for INSS tumor stage (stage 1, 2, 3, 4, 4s), MYCN status (normal, amplified), survival (dead, alive), age at diagnosis (younger than 18 months, older than 18 months)*

GAM analysis was performed by R statistical programming, version 3.4.3, by using the following function: *'test = gam (miRNA_abundance in_serum ~ Stage + MYCN_Status + OS_event + Age_cutoff, family = Gaussian (link = "identity"), method = "GCV.Cp", data = clinical_data)'*

For evaluation of the significance of miRNA abundance increase in murine serum in function of time, linear mixed-effects model analysis was used, assuming random effects: *'test = lme (miRNA_abundance_in_serum ~ timepoint, random = ~ 1/id, data = data, na.action = na.omit,)'.*

In order to evaluate the increase in hazard ratio in function of miRNAs levels in serum, the following Cox proportional hazards regression model was applied: *'test = coxph (Surv (survival time, event) ~ miRNA_abundance_in_serum, data = clinical_data)'* For comparison of median survival times between patients with low and high abundance of the metastatic disease markers in serum and for construction of Kaplan-Meier plots the following R-functions were applied respectively: *'overall_survival = survdiff (Surv (time,event) ~ expr.groups)'* and *'fit_KMplot = survfit (Surv (time,event) ~expr.groups)'.*

For data analysis of the serial neuroblastoma serum samples and control samples that were reprofiled, raw Cq data were analyzed using qbase+ version 3.1 (Biogazelle)[12]. Reference target stability was evaluated using geNorm analysis in qbase+; only 4 stably expressed reference miRNAs (miR-4755-5p, miR-4800-5p, miR-3909 and miR-766-3p) were retained for normalization. Log10 transformed normalized data were exported from qbase+ and used for further analysis in R.

**Results**

**Defining the human neuroblastoma circulating miRNome**

In order to define the neuroblastoma circulating miRNome, we created three serum pools, each composed of 5 serum samples (Figure 1). The pooled samples were screened for 1805 miRNAs. 751 well-expressed serum miRNAs, in at least one of the serum pools, were selected and subsequently used for expression analysis by RT-qPCR on 185 individual serum samples.

The serum samples were processed in 2 cohorts of 131 and 54 samples, respectively.

Eight miRNAs with missing values in more than 75% of the samples were excluded from the dataset. Normalization was performed on the resulting dataset, consisting of 185 samples and 743 miRNA expression values. All analyses were performed on the first cohort initially. The second cohort was used to validate the findings from the first cohort.

**Metastatic disease status has the largest impact on circulating miRNA levels in serum**

Generalized additive modeling was used on normalized miRNA expression values in order to quantify the association with 3 prognostic features (tumor stage, MYCN status, age at diagnosis) and overall survival status. A straightforward model was chosen, whereby interaction terms were excluded, since it was not the aim of the study to find the best fitted model for miRNA expression prediction, but to determine which of the disease features has the largest impact on serum miRNA expression and hence are responsible for the largest variance in the data set.

The analysis returned the level of significance (P-value) and the percentage of variance in the dataset explained by each of the tested features. The level of significance was used to measure the prediction ability of disease features for the 743 tested miRNAs. The percentage of variance was used to define the importance of each feature in influencing miRNA expression in serum. The average statistical significance, the number of significant miRNAs and the average variance explained by each disease feature is provided in Figure 2.

INSS tumor stage explains on average 33.1% of the variance in this serum miRNA dataset. Ten miRNAs were identified as being strongly significantly affected by tumor stage. The average level of significance was P-value < 0.0001 (range: $3.65 \times 10^{-2}$-$3.19 \times 10^{-11}$). Overall survival status (alive or dead of disease) explained an average 25.9% of the variance. Eight miRNAs were identified with an average significance of P-value < 0.05 (range: $3.58 \times 10^{-2}$-$5.88 \times 10^{-3}$). MYCN copy number status and age at diagnosis were responsible for 24.9% and 19.0% of the expression variance, respectively, with an average significance of P-value <0.01 and <0.05, respectively. The number of miRNAs to be significantly influenced by MYCN status or age at diagnosis were 4 and 3, respectively.

None of the identified miRNAs were significantly influenced by two or more disease characteristics. Based on the overall percentage of variance explained and the average level of significance for the identified sets of miRNAs for each disease feature, we concluded that INSS tumor stage had the largest impact on circulating serum miRNA levels.

**Identification of serum miRNAs as potential markers for disease burden**

Man-Whitney U test was used to select differentially expressed miRNAs between serum from patients with metastatic disease (stage 4 tumors) and serum from patients with localized disease (stage 1 and stage 2 tumors).

The analysis revealed 327 differentially expressed miRNAs (after correction for multiple-testing). 184 and 143 miRNAs were more abundant in serum from metastatic and localized disease patients, respectively (Figure 3a). The magnitude of the differences was much larger for miRNAs circulating in metastatic serum samples (Figure 3b). In fact, metastatic serum revealed 10 miRNAs with minimal fold-changes higher than 2 ∆Cq units (~4-fold difference), while the maximum fold change in serum from localized disease was lower than 2 ∆Cq units.

Interestingly, 9 out of the top 10 differential miRNAs were also put forward by the GAM analysis. These overlapping miRNAs were miR-873-3p, miR-149-5p, miR-124-3p, miR-218-5p, miR-490-5p, miR-323a-3p, miR-10b-3p, miR-375, and miR-129-5p (Figure 3c).

We further evaluated the serum levels of the 9 overlapping miRNAs in patients stratified according to INSS tumor stage. Average miRNA abundance was calculated per sample, samples were grouped per stage and depicted by a boxplot in Figure 3d.

A proportional increase of miRNA levels in serum from stage 1 to stage 4 patients was observed. Analysis of variance (Anova-test) was used to quantify the significance of the difference between stage groups. This analysis revealed a significant P-value of $2.0 \times 10^{-16}$. All differences were significant except for the differences between stage 1 and 2 tumors, and stage 4S and all other tumor stages.

Even though we would label the selected miRNAs as being indicators of metastatic tumor load (stage 4 disease) there is a discrepancy with respect to how the miRNAs behave in serum form stage 4S patients. Stage 4S patients have a particular pattern of metastatic disease with good outcome due to spontaneous regression; yet the expression of the selected set of 9 miRNAs is not significantly different from the localized tumor stages. This may be due to the fact that this cohort only contained a limited number of samples. Alternatively, it may reflect the difference in biology between stage 4 and 4S tumors, which may result in a different miRNA signature in patient serum samples. Based on these results, we assume that the identified set of miRNAs qualify as candidate markers of metastatic, non-stage 4S disease (hereafter referred to as markers for metastatic disease).

**Validation of serum markers for disease burden in independent patient cohort**

In order to evaluate the robustness of the identified markers for disease burden, we measured all 743 microRNAs in an independent cohort consisting of sera from 54 neuroblastoma patients.

There is a significantly positive correlation of localized versus metastatic (stage 4) fold change abundance between samples from the first and the second cohort (Pearson's r = 0.7, P<0.001). If only differential miRNAs between localized and metastatic disease are taken in to account, the correlation between the two independent sample cohorts is even higher (Pearson's r = 0.91, P<0.001).

In Figure 4a, a distribution of abundance fold changes is shown for localized versus metastatic disease. Seven of the 9 miRNAs identified on the first cohort have a four-fold abundance difference (2 log2 units) in the second sample cohort as well. The remaining two miRNAs show fold difference of more than 2.5 (1.4 log2 units).

A proportional increase of average miRNA expression from stage 1 to stage 4 was confirmed in the $2^{nd}$ cohort (Figure 4b). An ANOVA test revealed a significant of P-value of $5.8 \times 10^{-14}$. All differences between stage 4 samples and samples from other tumor stages were significant (P-value < 0.01). In Figure 4c, the ability of the set of 9 miRNA to classify the independent cohort into localized and metastatic disease is shown.

**Serum markers for disease burden are high but non-differential in primary tumors**

Two public datasets of miRNA expression in fresh-frozen diagnostic neuroblastoma tumors were used to evaluate expression of the serum markers for disease burden in the tumor. The tumor expression datasets were generated using different miRNA expression profiling technologies than used in the present study: (i) stem-loop RT-qPCR miRNA expression method (Life Technologies), as described in Mestdagh et al. (2008)[13] and (ii) small RNA sequencing as described in Schulte et al. (2010)[14].

There were 201 microRNAs measured in common between our serum dataset and the stem-loop RT-qPCR tumor dataset, including 6 of the 9 serum markers for disease burden. Fold changes were determined between 25 stage 4 tumors from patients with fatal outcome, and 25 stage 1 tumors from patients with favorable outcome (Figure 5a). No significant correlation was found between serum and tumor microRNA fold changes (Pearson = - 0.08, P-value = 0.22). Only 3 of the 6 miRNAs (miR-129-5p, miR-149-5p, and miR-323a3p) were significantly differentially expressed between metastatic and localized disease in the tumor dataset. However, the fold changes are in opposite direction as to what is observed in serum; i.e. while these miRNAs were more abundant in serum from metastatic disease, in tumor they were found to be higher expressed in localized disease. The 6 miRNAs were among the top 55% miRNAs with highest expression in the tumor RT-qPCR data (Figure 5c).

In the small RNA sequencing dataset, there were 5 patients with metastatic (stage 4) disease and fatal outcome and 5 patients with localized disease (stage 1) and favorable outcome. All 9 serum markers for metastatic disease were present in the tumor dataset. No significant correlation was found between serum and tumor microRNA fold changes (Pearson = - 0.13, P-value = 0.01). Only 2 of the 9 miRNAs (miR-129-5p and miR-149-5p) showed a significantly differential expression between metastatic and localized tumors (Figure 5b). Again, while in serum these miRNAs were more abundant in metastatic disease, in tumor they were higher expressed in localized disease. The 9 miRNAs were among the top 54% miRNAs with highest expression in the tumor sequencing data (Figure 5c).

**Serum microRNA markers for disease burden are specific for neuroblastoma**

We further evaluated the specificity of the selected set of 9 miRNAs in serum pools from healthy children and other pediatric cancer entities (sarcoma, nephroblastoma, and rhabdomyosarcoma), as depicted in Figure 6a and 6b. Low to absent levels were observed in healthy serum. When compared to serum from other pediatric cancer entities, expression levels for 8 out of 9 miRNAs were higher in neuroblastoma serum than in the pools from other pediatric cancer entities.

These results suggest that the increased levels of the identified set of miRNAs in serum from children with neuroblastoma is likely caused by the presence of neuroblastoma tumor cells in the patient's body.

**Serum miRNA markers for metastatic disease are strong predictors of overall survival in a global neuroblastoma patient cohort but not in the high-risk subgroup**

Given the importance of biomarkers for more accurate outcome prediction, the abundance of the 9 microRNAs in serum was assessed for potential association with overall survival. To this purpose Kaplan-Meier and Cox regression analyses were performed. The results showed a significant prediction of overall survival in a patient cohort including neuroblastoma patients from the low-, intermediate-, and the high-risk group (Table 2). However, when tested in the high-risk patient cohort only, none of the miRNAs was significantly associated with overall survival (Table 3).

Table 2: P-values and hazard ratios from Cox regression analysis testing for the relation of the 9 miRNA metastatic markers to overall survival, in the global neuroblastoma cohort (high-risk and non-high-risk patients, n = 185).

| miRNA | p-value | hazard ratio |
|---|---|---|
| hsa-miR-10b-3p | 1.1E-06 | 1.42 |
| hsa-miR-124-3p | 9.3E-04 | 1.20 |
| hsa-miR-129-5p | 4.0E-02 | 1.09 |
| hsa-miR-149-5p | 5.7E-02 | 1.14 |
| hsa-miR-218-5p | 7.9E-03 | 1.14 |
| hsa-miR-323a-3p | 4.1E-02 | 1.12 |
| hsa-miR-375 | 5.2E-04 | 1.16 |
| hsa-miR-490-5p | 4.7E-07 | 1.34 |
| hsa-miR-873-3p | 1.5E-05 | 1.45 |

Table 3: P-values and hazard ratios from Cox regression analysis testing for the relation of the 9 miRNA metastatic markers to overall survival of high-risk patients only (n = 106).

| miRNA | p-value | hazard ratio |
|---|---|---|
| hsa-miR-10b-3p | 0.27 | 1.10 |
| hsa-miR-124-3p | 0.84 | 1.02 |
| hsa-miR-129-5p | 0.83 | 1.01 |
| hsa-miR-149-5p | 0.53 | 1.05 |
| hsa-miR-218-5p | 0.49 | 1.04 |
| hsa-miR-323a-3p | 0.76 | 1.02 |
| hsa-miR-375 | 0.25 | 1.06 |
| hsa-miR-490-5p | 0.07 | 1.13 |
| hsa-miR-873-3p | 0.36 | 1.11 |

**Proportional increase of markers for disease burden in serum from mice engrafted with human neuroblastoma cells**

To evaluate to what extent tumor volume may impact the levels of miRNA markers for metastatic disease, we engrafted four-to-six-week-old immunodeficient mice with human luciferase-positive SH-SY5Y neuroblastoma cells. Small RNA sequencing was performed on serum samples collected 6 days before engraftment, and 11 days and 25 days after engraftment. Abundance levels of the selected set of 9 miRNAs were evaluated, in order to determine how the tumor graft impacted circulation of these miRNAs in murine serum. Significantly increased microRNA levels over time were observed for the selected miRNAs (Figure 7a). Of note, miR-873-3p, the only miRNA that is not conserved between human and mouse, was not detectable prior to engraftment and showed increasing levels 11 and 25 days post engraftment.

The higher detection levels of the selected miRNAs between 11 and 25 days post engraftment were associated with substantial tumor growth as evidenced by increased luciferase bioluminescence (Figure 7b).

These findings indicate that an increase in tumor volume could result in increased abundance of the 9 miRNAs in murine serum; at least one of the miRNAs (human specific miR-873-3p) must directly originate from the growing human tumor in the immunodeficient mice.

**Serum abundance of disease burden miRNA markers changes during treatment of metastatic neuroblastoma patients**

Subsequently, we set out to explore the use of the 9-miRNA signature to monitor disease burden during treatment of neuroblastoma patients diagnosed with metastatic disease. To this purpose, the abundance levels of the 9 miRNAs were measured using RT-qPCR in two serial serum samples from five stage 4 neuroblastoma patients, one collected at diagnosis (t0) and one during treatment (t1; Figure 8a; Table 4). Patients that demonstrated treatment response and presented with reduced tumor volume at time point t1 show a clear decrease in the average abundance of the 9 disease burden miRNA markers (Figure 8b). In contrast, abundance levels of these 9 miRNAs remain high or increase in non-responders, i.e. patients that show relapse or progressive disease at time point t1. These proof-of-concept data demonstrate that the identified 9-miRNA signature can potentially be used for disease monitoring in neuroblastoma.

EP 3 724 356 B1

Table 4: Clinico-pathological properties of the serial serum samples from neuroblastoma patients collected at diagnosis and during treatment.

| Patient ID | INSS stage | patient age at diagnosis (days) | MYCN amplification | serum sample annotation | serum collection days after diagnosis | overall survival status | follow-up days after diagnosis | relapse status | relapse days after diagnosis |
|---|---|---|---|---|---|---|---|---|---|
| P1 | 4 | 1136 | 0 | diagnostic (t0) | 0 | 1 | 670 | 1 | 571 |
| P1 | | | | treatment (t1) | 88 | | | | |
| P2 | 4 | 191 | | diagnostic (t0) | 0 | 1 | 324 | 1 | 170 |
| P2 | | | | treatment (t1) | 170 | | | | |
| P3 | 4 | 500 | 1 | diagnostic (t0) | 0 | 0 | 2302 | 0 | |
| P3 | | | | treatment (t1) | 194 | | | | |
| P4 | 4 | 997 | 1 | diagnostic (t0) | 0 | 1 | 111 | 1 | 89 |
| P4 | | | | treatment (t1) | 91 | | | | |
| P5 | 4 | 1316 | 0 | diagnostic (t0) | 0 | 1 | 332 | 1 | 260 |
| P5 | | | | treatment (t1) | 155 | | | | |

14

References

1. Cheung, N.-K. V. & Dyer, M. A. Neuroblastoma: developmental biology, cancer genomics and immunotherapy. Nat Cell Biol 13, 397-411 (2013).

2. Smith, M. A., Altekruse, S. F., Adamson, P. C., Reaman, G. H. & Seibel, N. L. Declining childhood and adolescent cancer mortality. Cancer 120, 2497-2506 (2014).

3. Hartomo, TB. et al. Minimal residual disease monitoring in neuroblastoma patients based on the expression of a set of real-time RT-PCR markers in tumor-initiating cells. Oncol Rep 29, 1629-1636 (2013).

4. Pugh, T. J. et al. The genetic landscape of high-risk neuroblastoma. Nat. Genet. 45, 279-284 (2013).

5. Blondal, T. et al. Assessing sample and miRNA profile quality in serum and plasma or other biofluids. Methods 59, S1-S6 (2013).

6. Murray, M. J. et al. Solid tumors of childhood display specific serum microRNA profiles. Cancer Epidemiol. Biomarkers Prev. 24, 350-360 (2015).

7. Ramraj, S. K. et al. Serum-circulating miRNAs predict neuroblastoma progression in mouse model of high-risk metastatic disease. Oncotarget 7, 18605-18619 (2016).

8. Mestdagh, P. et al. Evaluation of quantitative miRNA expression platforms in the microRNA quality control (mirQC) study. Nature Methods 8, 809-815 (2014).

9. Van Goethem, A. et al. Depletion of tRNA-halves enables effective small RNA sequencing of low-input murine serum samples. Sci. Rep. 1-11 (2016).

10. D'haene, B., Mestdagh, P., Hellemans, J. & Vandesompele, J. miRNA expression profiling: from reference genes to global mean normalization. Methods Mol. Biol. 822, 261-272 (2012).

11. Wood. Generalized Additive Models: An Introduction with R, Second Edition. Chapman & Hall/CRC Texts in Statistical Science. ISBN 9781498728331 (2017).

12. Hellemans et al. qBase relative quantification framework and software for management and automated analysis of real-time quantitative PCR data. Genome Biology. 8(2):R19 (2007).

13. Mestdagh, P. et al. High-throughput stem-loop RT-qPCR miRNA expression profiling using minute amounts of input RNA. Nucleic Acid Res. 36, e143 (2008).

14. Schulte, J. H. et al. Deep sequencing reveals differential expression of microRNAs in favorable versus unfavorable neuroblastoma. Nucleic Acids Research 38, 5919-5928 (2010).

## Claims

1. An in vitro method for assessing the disease burden in human neuroblastoma patients comprising:

   - providing a sample obtained from a patient,
   - quantifying the expression level of all of the following miRNAs: miR-124-3p, miR-375, miR-323a-3p, miR-129-5p, miR-218-5p, miR-490-5p, miR-149-5p, miR-873-3p and miR-10b-3p in said sample, and,
   - assessing the disease burden based on the quantity of the expression level of all of said miRNAs wherein a relative increased abundance of all of said miRNAs correlates with an increased disease burden, wherein said assessing the disease burden allows to monitor disease and to evaluate treatment response.

2. An in vitro method according to claim 1 wherein said sample obtained from a patient is a serum or a plasma sample.

3. An in vitro method according to claims 1-2 wherein said increased disease burden corresponds to stage 4 disease according to the International Neuroblastoma Staging System.

4. An in vitro method according to claims 1-3 wherein said quantifying the expression level of miRNAs occurs with a primer pair specific for each of the miRNAs for use in a PCR-based method.

5. An in vitro method according to claims 1-3 wherein said quantifying the expression level of miRNAs occurs with a probe specific for each of the miRNAs for use in a hybridization-based method.

6. An in vitro method according to claims 1-3 wherein said quantifying the expression level of miRNAs occurs via a sequence-based analysis of each of the miRNAs

**Patentansprüche**

1. In-vitro-Verfahren zur Beurteilung der Erkrankungsbelastung bei menschlichen Neuroblastompatienten, umfassend:

   - Bereitstellen einer aus einem Patienten erhaltenen Probe,
   - Quantifizierung des Expressionsniveaus aller der folgenden miRNAs: miR-124-3p, miR-375, miR-323a-3p, miR-129-5p, miR-218-5p, miR-490-5p, miR-149-5p, miR-873-3p und miR-10b-3p in der Probe, und
   - Beurteilung der Erkrankungsbelastung basierend auf der Größe des Expressionsniveaus aller dieser miRNAs, wobei eine relative erhöhte Häufigkeit aller dieser miRNAs mit einer erhöhten Erkrankungsbelastung korreliert, wobei die Beurteilung der Erkrankungsbelastung es ermöglicht, die Erkrankung zu überwachen und das Ansprechen auf eine Behandlung zu bewerten.

2. In-vitro-Verfahren nach Anspruch 1, wobei die aus einem Patienten erhaltene Probe eine Serum- oder Plasmaprobe ist.

3. In-vitro-Verfahren nach den Ansprüchen 1-2, wobei die erhöhte Erkrankungsbelastung einer Erkrankung des Stadiums 4 gemäß dem International Neuroblastoma Staging System entspricht.

4. In-vitro-Verfahren nach den Ansprüchen 1-3, wobei die Quantifizierung des Expressionsniveaus von miRNAs mit einem für jede der miRNAs spezifischen Primerpaar zur Verwendung in einem PCR-basierten Verfahren erfolgt.

5. In-vitro-Verfahren nach den Ansprüchen 1-3, wobei die Quantifizierung des Expressionsniveaus von miRNAs mit einer für jede der miRNAs spezifischen Sonde zur Verwendung in einem Hybridisierungs-basierten Verfahren erfolgt.

6. In-vitro-Verfahren nach den Ansprüchen 1-3, wobei die Quantifizierung des Expressionsniveaus von miRNAs mittels einer sequenzbasierten Analyse jeder der miRNAs erfolgt.

**Revendications**

1. Procédé *in vitro* pour l'évaluation de la charge de morbidité chez des patients humains atteints de neuroblastome comprenant :

   - la fourniture d'un échantillon obtenu d'un patient,
   - la quantification du niveau d'expression de tous les ARNmi suivants : miR-124-3p, miR-375, miR-323a-3p, miR-129-5p, miR-218-5p, miR-490-5p, miR-149-5p, miR-873-3p et miR-10b-3p dans ledit échantillon, et,
   - l'évaluation de la charge de morbidité sur la base de la quantité du niveau d'expression de tous lesdits ARNmi, une abondance augmentée relative de tous lesdits ARNmi corrélant avec une charge de morbidité augmentée, ladite évaluation de la charge de morbidité permettant de surveiller la maladie et d'évaluer une réponse à un traitement.

2. Procédé *in vitro* selon la revendication 1, ledit échantillon obtenu d'un patient étant un échantillon de sérum ou un échantillon de plasma.

3. Procédé *in vitro* selon les revendications 1 et 2, ladite charge de morbidité augmentée correspondant à une maladie de stade 4 selon le système international de classification des neuroblastomes.

4. Procédé *in vitro* selon les revendications 1 à 3, ladite quantification du niveau d'expression d'ARNmi ayant lieu avec une paire d'amorces spécifique pour chacun des ARNmi pour une utilisation dans un procédé à base de PCR.

5. Procédé *in vitro* selon les revendications 1 à 3, ladite quantification du niveau d'expression d'ARNmi ayant lieu avec sonde spécifique pour chacun des ARNmi pour une utilisation dans un procédé à base d'hybridation.

6. Procédé *in vitro* selon les revendications 1 à 3, ladite quantification du niveau d'expression d'ARNmi ayant lieu via une analyse à base de séquence de chacun des ARNmi.

Figure 1

Figure 2

|  | average variance (%) | average significance (-log₁₀ p) | number of miRNAs |
|---|---|---|---|
| INSS tumor stage | 33.1 | 4.4 | 10 |
| survival | 25.9 | 1.8 | 8 |
| MYCN status | 24.9 | 2.4 | 4 |
| age at diagnosis | 19.0 | 1.6 | 3 |

Figure 3

Figure 3

c

d

Figure 4

Figure 5

Figure 5

C

Figure 6

Figure 7

Figure 7

Figure 8

a

Figure 8

b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010066851 A

### Non-patent literature cited in the description

- **CHEUNG, N.-K. V. ; DYER, M. A.** Neuroblastoma: developmental biology, cancer genomics and immunotherapy. *Nat Cell Biol,* 2013, vol. 13, 397-411
- **SMITH, M. A. ; ALTEKRUSE, S. F. ; ADAMSON, P. C. ; REAMAN, G. H. ; SEIBEL, N. L.** Declining childhood and adolescent cancer mortality. *Cancer,* 2014, vol. 120, 2497-2506
- **HARTOMO, TB. et al.** Minimal residual disease monitoring in neuroblastoma patients based on the expression of a set of real-time RT-PCR markers in tumor-initiating cells. *Oncol Rep,* 2013, vol. 29, 1629-1636
- **PUGH, T. J. et al.** The genetic landscape of high-risk neuroblastoma. *Nat. Genet.,* 2013, vol. 45, 279-284
- **BLONDAL, T. et al.** Assessing sample and miRNA profile quality in serum and plasma or other biofluids. *Methods,* 2013, vol. 59, S1-S6
- **MURRAY, M. J. et al.** Solid tumors of childhood display specific serum microRNA profiles. *Cancer Epidemiol. Biomarkers Prev.,* 2015, vol. 24, 350-360
- **RAMRAJ, S. K. et al.** Serum-circulating miRNAs predict neuroblastoma progression in mouse model of high-risk metastatic disease. *Oncotarget,* 2016, vol. 7, 18605-18619
- **MESTDAGH, P. et al.** Evaluation of quantitative miRNA expression platforms in the microRNA quality control (mirQC) study. *Nature Methods,* 2014, vol. 8, 809-815
- **VAN GOETHEM, A. et al.** Depletion of tRNA-halves enables effective small RNA sequencing of low-input murine serum samples. *Sci. Rep.,* 2016, 1-11
- **D'HAENE, B. ; MESTDAGH, P. ; HELLEMANS, J. ; VANDESOMPELE, J.** miRNA expression profiling: from reference genes to global mean normalization. *Methods Mol. Biol.,* 2012, vol. 822, 261-272
- Generalized Additive Models: An Introduction with R. **WOOD.** Texts in Statistical Science. Chapman & Hall/CRC, 2017
- **HELLEMANS et al.** qBase relative quantification framework and software for management and automated analysis of real-time quantitative PCR data. *Genome Biology.,* 2007, vol. 8 (2), R19
- **MESTDAGH, P. et al.** High-throughput stem-loop RT-qPCR miRNA expression profiling using minute amounts of input RNA. *Nucleic Acid Res.,* 2008, vol. 36, e143
- **SCHULTE, J. H. et al.** Deep sequencing reveals differential expression of microRNAs in favorable versus unfavorable neuroblastoma. *Nucleic Acids Research,* 2010, vol. 38, 5919-5928